# EUROPEAN PATENT SPECIFICATION

(11) **EP 2 355 759 B1**
(45) Date of publication and mention of the grant of the patent: **23.11.2016**
(21) Application number: 09736794.0
(22) Date of filing: 08.09.2009
(51) Int. Cl.: A61F 9/00, A61M 5/32

(54) **THE EYE APPLICATOR FOR INJECTION APPLICATION OF SUBSTANCE INTO EYE TISSUE**
AUGENAPPLIKATOR ZUR INJIZIERUNG EINER SUBSTANZ IN AUGENGEWEBE
APPLICATEUR OPHTALMIQUE POUR UNE APPLICATION D'INJECTION DE SUBSTANCE DANS UN TISSU OCULAIRE

(30) Priority: 11.09.2008 CZ 20080559; 06.02.2009 CZ 20090066
(43) Date of publication of application: 17.08.2011
(73) Proprietor: Stodulka, Pavel, 76001 Zlín (CZ)
(72) Inventor: Stodulka, Pavel, 76001 Zlín (CZ)
(74) Representative: Kreizlova, Dana
(86) International application number: PCT/CZ2009/000107
(87) International publication number: WO 2010/028610

(56) References cited:
- WO-A-01/49226
- WO-A-2008/084063
- WO-A-2008/097072
- WO-A2-2008/098187
- US-A1- 2001 008 961
- US-A1- 2007 260 201
- US-B1- 6 579 265

## Description

### Technical field

The invention concerns an eye applicator for injection application of substance, especially medical or diagnostic liquid or liquid to refraction alter, into eye tissue, mainly into corneal tissue.

### Technical condition so far

A common syringe is currently used during surgeries for injecting medical or diagnostic liquid substances into eye tissues, especially into corneal tissue. For substance injection into larger areas, more incisions are needed to be done.

An eye applicator is know from WO 2008/097072 which relates to a device for intraocular administration of a liquid substance into human or animal eye by means of a hypodermic needle.

It is obvious that such a medical surgery is highly demanding on accuracy and a complication caused by partial or instantaneous imperfection for even experienced and skilled surgeons cannot be eliminated.

### Fundamentals of the invention

The invention is defined in claim 1. Preferred embodiments are defined in the dependent claims.

To a large extent, the eye applicator for injection application of substance into eye tissue helps to reduce and eliminate these problems according to the invention.

Fundamentals of the invention lie in the fact that the eye applicator is comprised of a main body, in which there is directly or by means of a segment one or more application injectors embedded, especially one or more application needles.

The main body can well have the shape of a cone or cylindrical ring with at least one side cutout. A segment in the form of a hollow body with a lateral area corresponding to the hollow formatively and with at least one frontal base with an openings is relatively embedded in the hollow of the main body. Inside the segment, there are at least two replaceable stirrup needle holders holding syringe needles on their inner curved frontal areas and on their lateral sides being equipped with thumbs nesting in curved grooves on the inner side of the segment base.

The main body of the applicator can also be shaped as a disc which is directly equipped with openings for needle insertion.

Small hollows of syringe needles attached onto the main body or stirrup holders of the needles are canals inside the main body or stirrup holders interconnected to the source of medical or diagnostic liquid substance. This interconnection may be realized in a way where the main body and/or stirrup holders are equipped with an opening for an external source of medical or diagnostic liquid substance access. Another possible solution lies in the fact that the main body and the stirrup holders have already built-in spaces for internal cartridges of medical or diagnostic liquid substance.

The main body of the eye applicator can well be on its surface equipped with ribbed holders and it can be made of transparent material.

The main benefit of the eye applicator according to the invention lies in the fact that it significantly reduces complication risks caused by potential inaccuracy of incision while injecting medical or diagnostic liquid substance in eye tissues, especially in corneal tissue.

### Brief description of the drawings

Attached drawings serve as further clarification of the fundamentals of the inventions; and wherein they introduce:
Fig. 1 - overall set of an exemplary design of an eye applicator
Fig. 2.1 - a detail of the main body of the eye applicator shown in an axonometric view
Fig. 2.2 - a detail of the main body of the eye applicator shown in a square projections
Fig. 3.1 - a detail of the segment of the eye applicator shown in an axonometric view
Fig. 3.2 - detail of the segment of the eye applicator shown in square projections
Fig. 4 - a detail of the stirrup needle holder shown in square projections
Fig. 5 - another exemplary design of an eye applicator.

### Examples of the invention designs

### Example 1

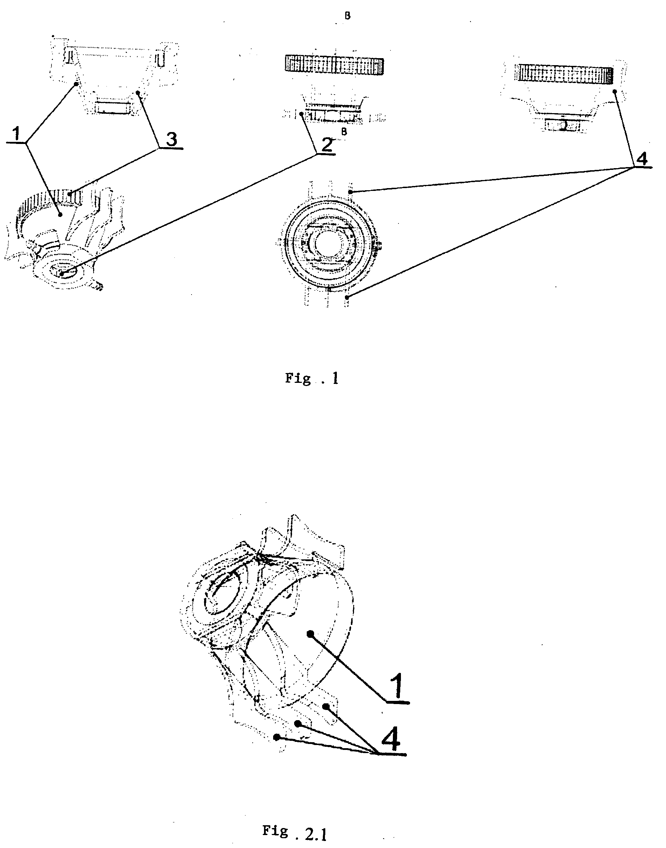

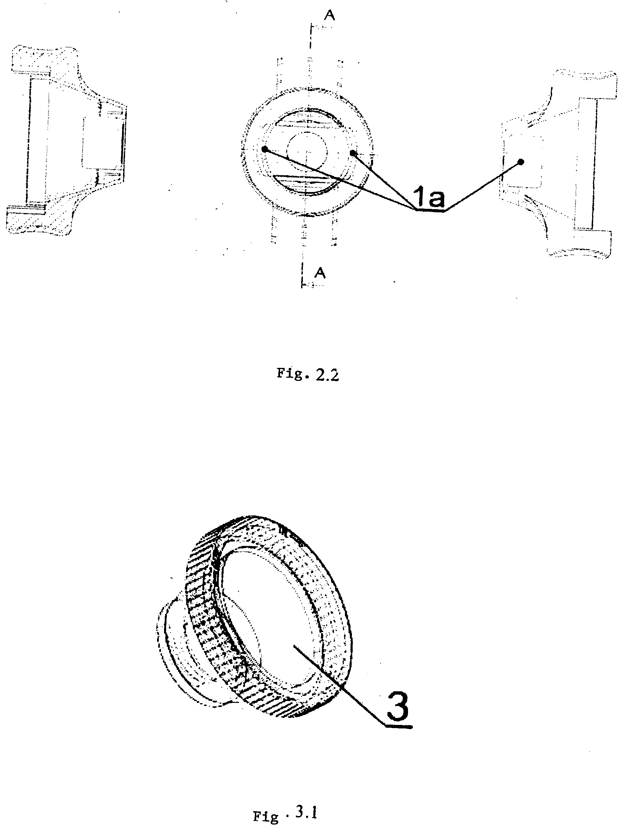

The eye applicator for injection of medical or diagnostic liquid substance in eye tissue in an exemplary design (see Fig. 1) is comprised of the main body (see Fig. 2) of a cone ring shape with two side cutouts 1a.

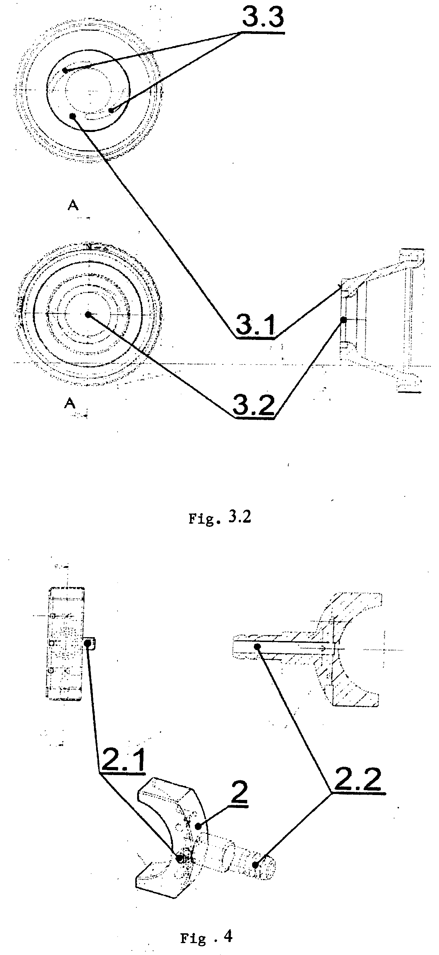

In the hollow of the body 1 is a segment 3 rotatively embedded (see Fig. 3), resolved as a hollow body with a lateral area corresponding formatively to this hollow, with a frontal base 3.1 with an opening 3.2.

Inside the segment 3 there are two replaceable stirrup needle holders 2 movingly embedded (see Fig. 4) holding syringe needles (not shown in Fig. 4) on their inner curved frontal areas, and on their lateral sides they are equipped with thumbs 2.1 nesting in curved grooves 3.3 on an inner side of the segment 3 base 3.1 (see Fig. 1, 3 and 4).

Small hollows of syringe needles attached onto the stirrup holders 2 of the needles are canals inside these stirrup holders 2 (not shown in Fig. 4) interconnected to the external source of medical or diagnostic liquid substance by the means of an opening 2.2.

While used, the applicator is placed to an eye and by turning the segment 3 centripetal movement of the stirrup needle holders 2 occurs and thus the needles are stabbed into the cornea. Consequently, medical or diagnostic liquid substance is compressively applied. By a subsequent turning back the segment 3 centrifugal movement of the stirrup needle holders 2 by which the needles are pushed out of the cornea. Finally, the applicator is removed from the eye.

### Example 2

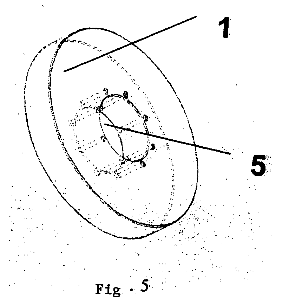

The eye applicator in another exemplary design (see Fig. 5) is comprised of the main body 1 in the shape of a disc and is made of transparent polycarbonate. This main body 1 is equipped with concetrically laid out openings 5 for needles insertion. Small hollows of the syringe needles embedded in the main body 1 are canals inside the body (not shown in the figure) interconnected to the source of medical or diagnostic liquid substance whereas the main body 1 has built-in spaces for internal cartridges of medical or diagnostic liquid substance.

## Claims

1. An eye applicator for an injection application of medical or diagnostic liquids and/or liquids for refraction alteration into corneal tissues, comprised of a main body (1) which is configured to contain one or more application needles, attachable to the main body (1) through a segment (3), whereby the main body (1) is a conical or cylindrical ring with at least one side cutout (1a), and the segment (3) being a hollow body has its outer surface shaped so as to correspond to the surface of a cavity in the main body (1), so that the segment (3) is able to turn inside said cavity, said Segment (3), having at least one base (3.1) with an opening (3.2), contains at least two replaceable needle holders (2), adapted to hold the application needles on their curved frontal surfaces and on their lateral sides being equipped with protrusions (2.1) nesting in arc-shaped grooves (3.3) which are created on the inner side of the base (3.1) of the segment (3), so that said needle holders (2) are attached by a shift able way to the segment (3). and can move in a centripetal or centrifugal direction when the segment is turned within the main body.

2. The eye applicator according to claim 1 wherein the needle holders are hollow and form canals that are configured to connect the application needles to a source of the liquid being applied.

3. The eye applicator according to claim 2, wherein the main body (1) or the needle holders (2) are equipped with openings (2.2) connectable to an external source of the liquid.

4. The eye applicator according to claim 2, wherein the main body (1) or the stirrup needle holders (2) have built-in spaces for internal cartridges with the liquid.

5. The eye applicator according to claim 1, wherein the main body (1) of the applicator is on its surface equipped with ribbed holders (4).

## Patentansprüche

1. Augenapplikator zur mittels Injizieren durchzuführenden Applikation medizinischer oder diagnostischer Flüssigkeiten und/oder Flüssigkeiten zur Refraktionsänderung in Hornhautgewebe, umfassend einen Hauptkörper (1), welcher konfiguriert ist, um eine oder mehrere Applikationsnadeln zu enthalten, welche durch ein Segment (3) an den Hauptkörper (1) angefügt werden können, wobei der Hauptkörper (1) ein konischer oder zylindrischer Ring ist mit mindestens einem Seitenausschnitt (1a), und das Segment (3) ein Hohlkörper ist, dessen Außenfläche so geformt ist, dass sie der Fläche eines Hohlraums in dem Hauptkörper (1) entspricht, so dass das Segment (3) innerhalb des genannten Hohlraums gedreht werden kann, und wobei das genannte Segment (3) über mindestens eine Basis (3.1) mit einer Öffnung (3.2) verfügt und mindestens zwei austauschbare Nadelhalter (2) enthält, die dazu angepasst sind, die Applikationsnadeln auf ihren gekrümmten Frontflächen zu halten, und die auf ihren lateralen Seiten Vorsprünge (2.1) aufweisen, welche in kreisbogenförmigen Nuten (3.3) verankert sind, welche an der Innenseite der Basis (3.1) des Segments (3) erzeugt wurden, so dass die genannten Nadelhalter (2) beweglich am Segment (3) befestigt sind und sich in zentripetaler und zentrifugaler Richtung bewegen können, wenn das Segment (3) im Hauptkörper (1) gedreht wird.

2. Augenapplikator nach Anspruch 1 **wobei** die Nadelhalter (2) hohl sind und Kanäle formen, welche konfiguriert sind, um die Applikationsnadeln mit einer Quelle der anzuwendenden Flüssigkeit zu verbinden.

3. Augenapplikator nach Anspruch 2, **wobei** der Hauptkörper (1) oder die Nadelhalter (2) Öffnungen (2.2) aufweisen, die mit einer externen Quelle der Flüssigkeit verbunden werden können.

4. Augenapplikator nach Anspruch 2, **wobei** der Hauptkörper (1) oder die Nadelhalter (2) über eingebaute Räume zur Aufnahme interner Patronen mit der Flüssigkeit verfügen.

5. Augenapplikator nach Anspruch 1, **wobei** der Hauptkörper (1) des Applikators auf seiner Oberfläche gerippte Halter (4) aufweist.

## Revendications

1. Un applicateur ophtalmique pour une application par injection de liquides médicaux ou de diagnostic, et/ou de liquides modifiant la réfraction dans les tissus cornéens, constitué du corps principal (1) conçu pour contenir une ou plusieurs aiguilles d'application, pouvant se fixer au corps principal (1) par l'intermédiaire d'un segment (3), le corps principal (1) étant un anneau conique ou cylindrique avec au moins une découpe latérale (la), et le segment (3) étant un corps creux dont la surface extérieure est profilée afin de correspondre à la surface d'une cavité dans le corps principal (1), pour que le segment (3) soit en mesure de tourner à l'intérieur de ladite cavité, le segment (3) ayant au moins une base (3.1) avec une ouverture (3.2), et contient au moins deux porte-aiguilles remplaçables (2) adaptés pour tenir les aiguilles d'application sur leurs surfaces frontales courbées et sur leurs côtés latéraux équipés de saillies (2.1) s'emboîtant dans les rainures en forme d'arc (3.3) usinées sur la face intérieure de la base (3.1) du segment (3), de sorte que lesdits porte-aiguilles (2) sont fixés en pouvant bouger au segment (3), avec la capacité à se déplacer dans la direction centripète ou centrifuge, lorsque le segment (3) est tourné dans le corps principal (1).

2. L'applicateur ophtalmique, conformément à la revendication 1, où les porte-aiguilles (2) sont creux et forment des canaux configurés pour connecter les aiguilles d'application à une source de liquide à appliquer.

3. L'applicateur ophtalmique, conformément à la revendication 2, où le corps principal (1) ou les porte-aiguilles (2) sont équipés d'ouvertures (2.2) pouvant être connectées à une source externe de liquide.

4. L'applicateur ophtalmique, conformément à la revendication 2, où le corps principal (1) ou les porte-aiguilles (2) sont équipés d'espaces intégrés pour cartouches internes de liquide.

5. L'applicateur ophtalmique, conformément à la revendication 1, où le corps principal (1) de l'applicateur est équipé à sa surface de supports nervurés (4).
